# EUROPEAN PATENT APPLICATION

(11) **EP 4 299 579 A1**
(43) Date of publication of application: **03.01.2024**
(21) Application number: 23181408.8
(22) Date of filing: 26.06.2023
(51) Int. Cl.: C07H 19/01

(54) **METHOD FOR PRODUCING LEVOGLUCOSAN**

(30) Priority: 30.06.2022 JP 2022105433; 15.06.2023 JP 2023098562
(71) Applicant: Kyoto University, Kyoto-shi, Kyoto 606-8501 (JP); Shin-Etsu Chemical Co., Ltd., Tokyo (JP)
(72) Inventor: KAWAMOTO, Haruo, Kyoto-shi (JP); GINTING, Resi Vita Loka, Kyoto-shi (JP); MATSUO, Kana, Kyoto-shi (JP); NOMURA, Takashi, Kyoto-shi (JP); MARUICHI, Yasuko, Kyoto-shi (JP); MINAMI, Eiji, Kyoto-shi (JP); KOBAYASHI, Kazuto, Chiyoda-ku (JP); MIKI, Akiko, Chiyoda-ku (JP)
(74) Representative: Mewburn Ellis LLP

(57) **Abstract**

A method is provided for the easy industrial production of levoglucosan in a high yield and at high purity using pulp as the feedstock. The method includes:
(1) immersion-treating pulp having a content of glucose as a constituent sugar of at least 80 wt% and a Klason lignin content of not more than 1 wt% in an aqueous solution of a divalent metal salt;
(2) carrying out heat treatment by heating and thereby pyrolyzing the immersion-treated pulp to form gaseous levoglucosan and also to either not form char or form a film-like char and to either not form carbon monoxide or form not more than 5 parts by weight of carbon monoxide per 100 parts by weight (bone-dry weight) of the pulp; and
(3) recovering the gaseous levoglucosan by cooling it to a temperature at or below the boiling point of levoglucosan.

## Description

### TECHNICAL FIELD

The present invention relates to a method for producing levoglucosan.

### BACKGROUND

Cellulosic biomass from plant sources such as wood and cotton is the most abundant and readily accessible biomass resource on Earth. Being inedible, cellulosic biomass has been chiefly used historically as fuel, construction materials, textile fibers, paper and base polymers for semisynthetic polymers. Recently, through use by conversion to chemical substances that can serve as renewable resources for supplanting fossil fuel resources such as petroleum and natural gas, cellulosic biomass is expected to play a role in slowing the rise in atmospheric carbon dioxide regarded as a major cause of global warming. However, the technology for doing so has yet to be fully developed.

Cellulose is a natural polymer in which β-glucose units are linearly polymerized. The sugars and their anhydrides (sugar anhydrides) obtained by cellulose conversion are useful chemical substrates which can be utilized as, for example, feedstocks in the synthesis of pharmaceuticals. If it were possible to economically and practically convert inedible cellulosic biomass into these chemical substances, the significance of doing so would be enormous, not only because such biomass could then be employed in place of the edible resources such as grains which, although inherently useful as foods, are being used to obtain such chemical substances, but also from the standpoint of the food supply for the human population which continues today to grow.

One such conversion technology is cellulosic biomass saccharification by way of known methods such as acid hydrolysis, the sub/supercritical water process and pyrolysis. The practical application of acid hydrolysis and the sub/supercritical water process poses challenges in terms of, for example, waste acid treatment, the harsh reaction conditions, and the difficulty of increasing the concentration of the sugar solution.

On the other hand, pyrolysis, which is a process for obtaining sugars by employing a reusable solid acid catalyst to hydrolyze sugar anhydrides such as levoglucosan that form as a result of thermal degradation, requires no waste acid treatment and has the additional advantage that, when carried out under dry conditions, increasing the concentration of the sugar solution is easy. Moreover, the high-concentration sugar solution obtained as a result can serve as a good fermentation feedstock.

However, in the pyrolysis process, due to thermal degradation, various volatile products such furfurals and also carbonized matter form as by-products due to thermal degradation. In addition, the sugar anhydrides that have formed undergo further decomposition in a gas phase, fragmenting into smaller molecules such as carbon monoxide, carbon dioxide, methane and ethylene, and undergo polymerization in a liquid phase, causing secondary decomposition to arise and thus making it difficult to obtain the sugar anhydrides in a high yield. Also, because the aldehydes and furfurals that form as by-products are fermentation inhibitors, they exert an influence on subsequent fermentation steps as well.

Methods for producing sugar anhydrides by the pyrolysis of cellulosic biomass which address this issue have been described in the literature. For example, one method produces levoglucosan (1,6-anhydro-(β-D-glucopyranose) by heating, at a temperature between 300°C and 500°C in a tubular furnace under reduced pressure or a stream of nitrogen, a cellulose-containing material from which inorganics have been removed by pickling (J. Appl. Polym. Sci. (1979), 23, pp. 3525-3539). Another method produces levoglucosan by heating, at a temperature of between 300°C and 350°C under reduced pressure and a stream of nitrogen for a period of 20 to 60 minutes, wood and newsprint from which inorganics have been removed by pickling and into which metal ions have been introduced by ion exchange (J. Anal. Appl. Pyrolysis (1991), 21, pp. 133-146). This report proposes, as the mechanism behind the increase in levoglucosan yield, the hypothesis that the metal ions reduce the inhibition of levoglucosan formation by lignin.

However, the tar recovered as the product in J. Appl. Polym. Sci. (1979) had a levoglucosan concentration of from 30 to 68 wt%. Also, in J. Anal. Appl. Pyrolysis (1991), when divalent iron ions were introduced, under which conditions the levoglucosan yield was at its highest, the levoglucosan-containing tar yield was 50 wt% (feedstock basis) and the levoglucosan yield was 15.8 wt% (feedstock basis); the levoglucosan concentration in the tar was thus 31.6 wt%. When the levoglucosan obtained under these conditions is used as a fermentation feedstock, purification operations for removing the aldehydes and furfurals that are fermentation inhibitors are required. Hence, there exists today a desire for an improvement in the levoglucosan concentration of the recovered product.

Moreover, when one considers the fact that, in J. Anal. Appl. Pyrolysis (1991), using cellulose as the feedstock, the maximum levoglucosan yield before purification is only 32 wt% (based on the cellulose in the feedstock) and the yield after purification decreases even more, a further improvement in yield is desired.

Furthermore, in J. Appl. Polym. Sci. (1979), high-purity, high-cost cellulose such as filter paper, microcrystalline cellulose and cotton linter is used as the feedstock, which is hardly ideal for industrializing levoglucosan production.

### SUMMARY OF THE INVENTION

In light of the above circumstances, the object of this invention to provide an easy method for the industrial production of levoglucosan in a high yield and at high purity using pulp as the feedstock.

We have conducted intensive investigations in order to resolve the above problems. As a result, we have discovered that by using pulp having a content of glucose as a constituent sugar of at least 80 wt% and a Klason lignin content of not more than 1 wt%, immersion treating the pulp in an aqueous solution of a divalent metal salt and then subjecting it to pyrolysis, levoglucosan can be easily obtained in a high yield and at high purity.

Accordingly, the present invention provides a method for producing levoglucosan, which method includes the steps of:
(1) immersion-treating pulp having a content of glucose as a constituent sugar of at least 80 wt% and a Klason lignin content of not more than 1 wt% in an aqueous solution of a divalent metal salt;
(2) carrying out heat treatment by heating and thereby pyrolyzing the immersion-treated pulp to form gaseous levoglucosan and also to either not form char or form a film-like char and to either not form carbon monoxide or form not more than 5 parts by weight of carbon monoxide per 100 parts by weight (bone-dry weight) of the pulp; and
(3) recovering the gaseous levoglucosan by cooling it to a temperature at or below the boiling point of levoglucosan.

In a preferred embodiment of the method for producing levoglucosan of the invention, the pulp has a content of xylose as a constituent sugar of from 5 to 20 wt%.

In another preferred embodiment of the inventive method, the pulp has a content of glucose as a constituent sugar of not more than 95 wt%.

In yet another preferred embodiment, the divalent metal salt is a magnesium salt.

In still another preferred embodiment, the pulp prior to immersion treatment has not been desalted.

In a further preferred embodiment, the heat treatment is heating by infrared irradiation.

In a yet further preferred embodiment, the pulp heating time in heat treatment is at most 60 seconds.

In a still further preferred embodiment, the pulp itself has a temperature in the heat treatment step which is between 400°C and 450°C.

### ADVANTAGEOUS EFFECTS

This invention makes it possible to industrially produce levoglucosan from pulp in a high yield and at high purity by immersion-treating the pulp in an aqueous solution of a divalent metal salt and subsequently pyrolyzing the immersion-treated pulp. Moreover, relatively low-purity, low-cost pulp having a glucose content among the constituent sugars of from 80 to 95 wt% can be used. In addition, heat treatment does not require a special apparatus. For example, pyrolysis can be completed in a short time of 60 seconds or less under normal pressure.

### BRIEF DESCRIPTION OF THE DIAGRAM

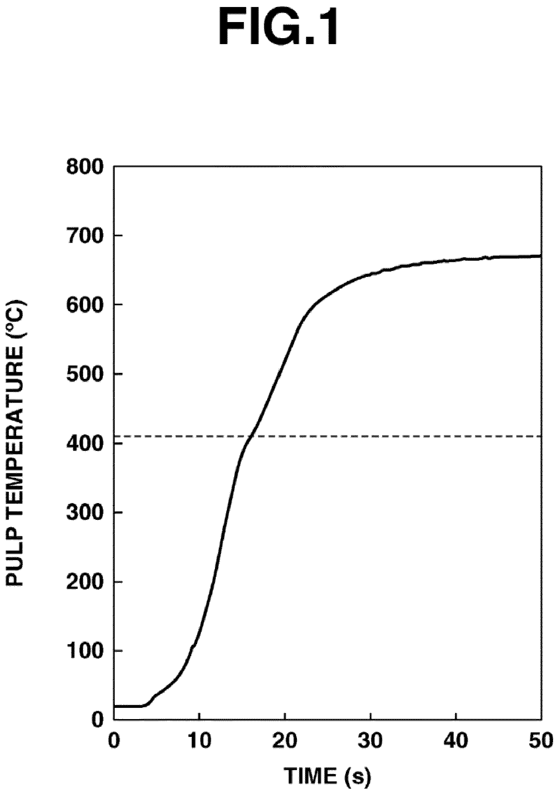

FIG. 1 is a temperature rise chart showing the results of pulp temperature measurements during the heat treatment of bleached pulp 3 following immersion treatment.

### FURTHER EXPLANATIONS; OPTIONS AND PREFERENCES

The objects, features and advantages of the invention will become more apparent from the following detailed description.

The inventive method for producing levoglucosan includes the steps of:
(1) immersion-treating pulp having a content of glucose as a constituent sugar of at least 80 wt% and a content of Klason lignin of not more than 1 wt% in an aqueous solution of a divalent metal salt (immersion step);
(2) carrying out heat treatment by heating and thereby pyrolyzing the immersion-treated pulp to form gaseous levoglucosan (1,6-anhydro-(β-D-glucopyranose) and also to either not form char (carbonized solid residue) or form a film-like char and to either not form carbon monoxide or form not more than 5 parts by weight of carbon monoxide per 100 parts by weight (bone-dry weight) of the pulp (heating step); and
(3) recovering the gaseous levoglucosan by cooling it to a temperature at or below the boiling point of levoglucosan.

### Step (1): Immersion Step

The immersion step carries out immersion treatment in which pulp having a content of glucose as a constituent sugar of at least 80 wt% and a Klason lignin content of not more than 1 wt% is immersed in an aqueous solution of a divalent metal salt, causing divalent metal ions from the divalent metal salt to form coordination bonds with functional groups (carboxyl groups) on the pulp. Pulp may generally include cellulose, hemicellulose and lignin. Here, "Klason lignin" refers to the acid-insoluble lignin obtained as a residue after cellulose and hemicellulose are removed from pulp by two-stage sulfuric acid treatment as subsequently described.

We have found that, compared with monovalent metal ions, divalent metal ions form coordination bonds more easily with the carboxyl groups in pulp. Therefore, even if the pulp prior to immersion treatment contains monovalent metal ions, immersion treatment may be performed without carrying out desalting treatment to remove the monovalent metal ions. However, prior to the immersion step, sodium and other monovalent metal ions contained within the pulp may be optionally removed by carrying out desalting treatment in which the pulp is immersed in an acidic aqueous solution such as dilute hydrochloric acid and washed with deionized water or the like.

The above pulp is not particularly limited, so long as the content of glucose as a constituent sugar is at least 80 wt% and the content of Klason lignin is not more than 1 wt%. However, chemical pulp is preferred on account of its low Klason lignin content. The pulp raw material is not particularly limited, and may be either wood or non-wood. Examples include softwoods and hardwoods. Because a large amount of carboxyl groups in the pulp is desirable for obtaining levoglucosan in a high yield and at high purity, hardwoods are preferred. Hardwoods generally have a higher xylan content than softwoods, and xylan contains more carboxyl groups than other hemicellulose components. The pulp digestion process also is not subject to any particular limitations; examples include the kraft process and the sulfite process. The kraft process is preferred because of the absence of sulfonic acid, which is a major factor in the formation of levoglucosenone as a by-product. A single type of pulp may be used alone or two more types may be used in combination.

The glucose content within the constituent sugars is at least 80 wt%, preferably at least 81 wt%, and more preferably at least 83 wt%, and is preferably not more than 95 wt%, more preferably not more than 93 wt%, and most preferably not more than 90 wt%.

The constituent sugars other than glucose include, without particular limitation, xylose, mannose, galactose and arabinose. Given that a larger amount of carboxyl groups in the pulp is better for obtaining levoglucosan in a high yield and at high purity, the xylose content as a constituent sugar is preferably from 5 to 20 wt%. Xylan, which is largely composed of xylose, generally contains more carboxyl groups than other hemicellulose components. In addition, it is more preferable for the content of mannose as a constituent sugar to be from 0 to 3 wt%.

Determination of Constituent Sugars (Glucose, Xylose and Mannose) and Klason Lignin:
After the pulp to be measured has been subjected to acid hydrolysis treatment, the constituent sugars of the pulp are quantitatively determined by ion chromatography. Separately, the Klason lignin is quantitatively determined from the amount of acid hydrolysis treatment residue. This may be carried out as follows.

A predetermined amount of pulp is immersed for 2 hours in 72 wt% sulfuric acid (liquid temperature, 25°C), following which water is added, diluting the liquid to a sulfuric acid concentration of 3 wt%, and 30 minutes of heating at 121°C is carried out. The treated material is then filtered, separating it into an acid hydrolysis liquor and residue, following which the acid hydrolysis liquor is neutralized and the constituent sugars in the pulp are quantitatively determined by ion chromatography under the following conditions.

| | |
|---|---|
| • Apparatus: | Prominence Ion Analysis System LC-20ADsp (Shimadzu Corporation) |
| • Column: | CarboPac^{™} PA1 (4 × 250 mm, Thermo Scientific, Waltham, MA, USA) |
| • Detector: | DECADE Elite (Antec Scientific) |
| • Mobile phase: | 0.03 mol/L aqueous sodium hydroxide solution |
| • Flow rate: | 1.0 mL/min |
| • Column temperature: | 35°C |

Separately, the residue is dried within a thermostatic chamber at 105°C and the constant weight of the residue is determined as the Klason lignin.

The Klason lignin content is not more than 1 wt%, and preferably not more than 0.5 wt%. By setting the Klason lignin content to not more than 1 wt%, levoglucosan can be industrially produced from this pulp in a high yield and at high purity. When Klason lignin remains behind, guaiacols and other Klason lignin hydrolysates end up in the product, which may lower the levoglucosan purity within the product.

An example of pulp having a Klason lignin content of not more than 1 wt% is bleached pulp obtained by carrying out bleaching treatment on chemical pulp. In this case, the content of Klason lignin within the bleached pulp obtained by carrying out bleaching treatment on chemical pulp is normally 1 wt% or less.

The form of the pulp is not particularly limited, and may be, for example, a sheet, powder, fibers or particles. A pulp in sheet form is preferable because grinding and fibrillating operations are not required.

Because a large amount of carboxyl groups in the pulp is desirable for obtaining levoglucosan in a high yield and at high purity, the saturation metal content of the pulp is preferably from 5 to 100 µmol/g, and more preferably from 10 to 50 µmol/g.

Saturation Metal Content of Pulp:
Given the fact that the functional groups within the pulp which form salts with metal ions are carboxyl groups and that sodium ions form salts in a one-to-one ratio with carboxyl groups, the amount of carboxyl groups in the pulp can be assumed to be equal to the total amount of metal ions included in the pulp following immersion treatment using an aqueous solution of a monovalent metal salt (which amount is referred to below as the "saturation metal content of the pulp"). Here, the "saturation metal content of the pulp" is the total amount of sodium ions, potassium ions, magnesium ions and calcium ions included in the pulp obtained by taking the pulp prior to immersion treatment in this step and immersing it for a given length of time (e.g., 18 hours) in an aqueous solution of a monovalent metal salt (solution temperature, 25°C), such as an aqueous sodium acetate solution or an aqueous potassium acetate solution having a given concentration, then washing the pulp with deionized water, drying the washed pulp, returning it to normal temperature (20°C) and air drying.

Examples of the divalent metal salt making up the above aqueous solution of a divalent metal salt include magnesium salts such as magnesium acetate, magnesium sulfate, magnesium nitrate, magnesium chloride, magnesium bromide and magnesium iodide; calcium salts such as calcium acetate, calcium nitrate, calcium chloride, calcium bromide and calcium iodide; manganese(II) salts such as manganese acetate, manganese sulfate, manganese nitrate, manganese chloride, manganese bromide and manganese iodide; iron(II) salts such as iron sulfate, iron nitrate, iron chloride, iron bromide and iron iodide; cobalt(II) salts such as cobalt acetate, cobalt sulfate, cobalt chloride, cobalt bromide and cobalt iodide; nickel(II) salts such as nickel acetate, nickel sulfate, nickel nitrate, nickel chloride, nickel bromide and nickel iodide; and copper(II) salts such as copper sulfate, copper nitrate, copper chloride and copper bromide. From the standpoint of obtaining levoglucosan in a high yield and at high purity, a magnesium salt or a calcium salt is preferred; a magnesium salt is more preferred. Magnesium acetate or calcium acetate are even more preferred, and magnesium acetate is most preferred. A single divalent metal salt may be used alone, or two or more may be used in combination.

The concentration of the aqueous divalent metal salt solution may be, for example, from 0.01 to 1 mol/L, so long as it is a sufficient concentration for causing the divalent metal ions to form coordination bonds with carboxyl groups in the pulp.

The method of immersing the pulp in the aqueous divalent metal salt solution is not particularly limited, so long as it is a method that can cause the divalent metal ions to form coordinate bonds with carboxyl groups in the pulp. The pulp may be immersed in an aqueous divalent metal salt solution, or the pulp may be immersed in water, following which a divalent metal salt may be added.

The immersion temperature in immersion treatment (temperature of aqueous divalent metal salt solution) is not particularly limited, so long as the divalent metal ions can be made to form coordination bonds with carboxyl groups in the pulp. For example, the temperature may be between 5°C and 100°C, preferably between 10°C and 70°C, and more preferably between 15°C and 40°C.

The immersion time in immersion treatment, so long as it is a time sufficient to enable the divalent metal ions to form coordination bonds with carboxyl groups in the pulp, may be, for example, 1 hour or more, preferably 2 hours or more, and more preferably 4 hours or more. Although there is no particular limit in the immersion time, the length of treatment may be, for example, 50 hours or less, and preferably 24 hours or less. At an immersion time of less than 1 hour, it may not be possible for the divalent metal ions to sufficiently form coordination bonds with carboxyl groups in the pulp.

Following immersion treatment, the pulp may be optionally washed with deionized water or the like to remove divalent metal ions that have not formed coordination bonds with carboxyl groups in the pulp, and then dried.

Although there is no particular upper limit in the amount of divalent metal salt in the aqueous divalent metal salt solution relative to the amount of carboxyl groups in the pulp, at less than a 3-fold amount, the divalent metal ions may not be fully able to form coordination bonds with carboxyl groups in the pulp and so the advantageous effects of the invention may not be achieved.

To obtain levoglucosan in a high yield and at high purity, the amount of divalent metal ions present in the pulp following immersion treatment is preferably from 5 to 100 µmol/g, and more preferably from 7 to 50 µmol/g.

The amount of monovalent metal ions contained in the pulp following immersion treatment is preferably not more than 5 µmol/g, and more preferably not more than 2 µmol/g. Monovalent metal ions give rise to the fragmentation of xylan in heat treatment (J. Anal. Appl. Pyrolysis (2018), 136, pp. 215-221). Hence, when the amount of monovalent metal ions exceeds 5 µmol/g, xylan fragmentation may arise and the stability of the pulp to heat in the course of temperature ramp-up may decrease. In addition, monovalent metal ions may decompose the levoglucosan that has formed in heat treatment.

### Step (2): Heating Step

The heating step carries out heat treatment by heating and thereby pyrolyzing the immersion-treated pulp to form gaseous levoglucosan and also to either not form char or form a film-like char and to either not form carbon monoxide or form not more than 5 parts by weight of carbon monoxide per 100 parts by weight (bone-dry weight) of the pulp.

The pulp itself has a temperature in heat treatment which is preferably between 400°C and 500°C, and more preferably between 400°C and 450°C.

Here, the temperature of the heated pulp in the heating step can be estimated from the presence or absence of char (carbonized solid residue) formation and, in cases where char forms, the form of such char, and also from the gas composition (specifically, the presence or absence of carbon monoxide formation and, in cases where carbon monoxide forms, the amount of such formation). When the temperature of the pulp itself is less than 400°C, the char retains the form of the original pulp, but when the temperature of the pulp is 400°C or more, either char does not form or, if it does form, it liquefies under heating and then becomes a film-like char (J. Anal. Appl. Pyrolysis (2014), 109, pp. 185-195). When the temperature of the pulp is greater than 500°C, the levoglucosan that forms due to pyrolysis gives rise to further fragmentation, forming primarily carbon monoxide (ChemSusChem (2015), 8, pp. 2240-2249). The amount of carbon monoxide formation at this time comes to exceed 5 parts by weight per 100 parts by weight of the bone-dry weight of the pulp.

Therefore, when the immersion-treated pulp has been heat-treated, if char does not form or a film-like char forms and if carbon monoxide does not form or, if it does form, the amount of such formation is not more than 5 parts by weight per 100 parts by weight of the bone-dry weight of the pulp, the temperature of the pulp at this time is estimated to be between 400°C and 500°C.

The amount of carbon dioxide formation per 100 parts by weight of the bone-dry weight of the pulp is preferably not more than 3 parts by weight, more preferably not more than 1 part by weight, even more preferably not more than 0.5 part by weight, and most preferably 0 part by weight.

In cases where the temperature of the pulp following heating is estimated to be less than 400°C, heating is inadequate and some of the pulp may not pyrolyze, the selectivity of the pyrolysis reaction may decrease, resulting in an increase in by-product formation, or the levoglucosan that has formed may liquefy and polymerize. Similarly, even in the course of temperature ramp-up to a pulp temperature of 400°C in heat treatment, low-selectivity pyrolysis reactions may arise, leading to the formation of by-products; hence, it is preferable for the pulp to have a high stability to heat in the course of temperature ramp-up. On the other hand, in cases where the temperature of the pulp after heating is estimated to exceed 500°C, the levoglucosan that has formed sometimes undergoes fragmentation, decomposing to smaller molecules such as carbon monoxide, carbon dioxide, methane and ethylene. The temperature of the pulp can be directly measured by a known method such as with thermocouples, infrared radiation thermometers or the like.

We have confirmed from our own investigations that, compared with generally available chemical pulp, i.e., pulp that contains monovalent metal ions, the above immersion-treated pulp has a high stability to the heat of the pulp in the course of temperature ramp-up, suppressing the formation of by-products due to low-selectivity pyrolysis reactions. In addition, we have confirmed that the weight loss temperature region in thermogravimetry is about 50°C lower for this immersion treated pulp than for pulp containing monovalent metal ions. The reason appears to be that, owing to Lewis acid catalysis by divalent metals, pyrolysis at low temperatures is promoted relative to pulp containing monovalent metal ions. Also, of commonly available chemical pulps, those pulps which use monovalent metal ion-containing chemicals in digestion, such as kraft pulp, contain monovalent metal ions.

That is, in the practice of this invention, owing to the above-described immersion treatment in an aqueous divalent metal salt solution prior to heat treatment, in a heating step at between 400 to 450°C, pyrolysis of the pulp can be promoted while suppressing both low-selectivity pyrolysis reactions in the course of temperature ramp-up and the formation of carbon monoxide at 500°C and above, enabling levoglucosan to be obtained in a high yield and at high purity.

To prevent a decrease in the selectivity of the pulp pyrolysis reactions and a resulting increase in by-products and to keep the levoglucosan that has formed from liquefying and polymerizing, the temperature ramp-up rate in the heating step is preferably at least 10°C/s, more preferably at least 30°C/s, and even more preferably at least 50°C/s. Although there is no particular upper limit in the ramp-up rate, from the standpoint of handleability, it is desirable for the ramp-up rate to be not more than 5,000°C/s.

In the heating step, from the standpoint of the production efficiency of the target product and in order to minimize any decrease in yield from secondary decomposition due to reactions at low temperature and in the superheated region, the pulp heating time (i.e., the heating hold time after the pulp temperature has reached a temperature estimated to be between 400°C and 500°C, preferably between 400°C and 450°C) is preferably at most 60 seconds, more preferably not more than 30 seconds, even more preferably not more than 15 seconds, and even more preferably not more than 10 seconds.

No particular limitation is imposed on the heating method used in the heating step. Illustrative examples include methods of heating the pulp indirectly by infrared or microwave irradiation, methods of heating the pulp by contacting it with a high-temperature gas such as air or nitrogen in a fluidized bed or the like, methods of heating the pulp by bringing it into direct contact with a heated jacket wall or agitator paddles in an agitating tank, and combinations of these methods. From the standpoint of the ability to rapidly cool the gaseous levoglucosan that has formed so as to keep the surrounding atmosphere from heating up and thereby suppressing the secondary decomposition of levoglucosan, a method of indirect heating by infrared irradiation is preferred.

The heating step may be a batch process which feeds a fixed amount of the starting pulp into a heating apparatus, subjects it to heat treatment and recovers the product, or may be a continuous process which feeds the starting pulp continuously into a heating apparatus and continuously recovers the product.

The atmosphere within the heating apparatus that carries out heat treatment is not limited to atmospheric pressure. From the standpoint of optimal process design, an inert gas atmosphere or one that is under reduced or applied pressure is also possible.

The levoglucosan obtained by the heating step is normally gaseous.

### Step (3): Recovery Step

The recovery step recovers the gaseous levoglucosan obtained from the heating step by cooling it to its boiling point (385°C) or below. The levoglucosan that has formed gives rise to secondary decomposition at about 400°C and below. Hence, by rapidly cooling the levoglucosan down to 100°C or below at which it stably exists, secondary decomposition of the levoglucosan is minimized, enabling the yield of levoglucosan as the target substance to be increased.

The method of recovery is not particular limited, so long as it can cool gaseous levoglucosan down to its boiling point or below. Illustrative examples include a recovery method that rapidly mixes low-temperature air or nitrogen with the gaseous levoglucosan-containing high-temperature gas stream, thereby cooling it and rendering it into the form of an aerosol, a recovery method that brings the gaseous levoglucosan into contact with a low-temperature liquid in a scrubber or the like and dissolves it, and a recovery method that brings the gaseous levoglucosan into contact with a low-temperature solid, converting it into a liquid or solid state.

The cooling temperature is preferably 300°C or less, more preferably 200°C or less, and even more preferably 100°C or less.

As mentioned above, the inventive method for producing levoglucosan enables levoglucosan to be industrially produced from pulp in a high yield and at high purity simply by, as a treatment preliminary to heat treatment, immersing the pulp in an aqueous divalent metal salt solution, and then pyrolyzing the pulp. This is possible even when low-cost, relatively low-purity pulp having a content of glucose within the constituent sugars of from 80 to 95 wt% is used as the starting material. Also, heat treatment does not require special equipment; for example, pyrolysis can be completed in a short time of 60 seconds or less under normal pressure.

### EXAMPLES

The invention is illustrated more fully below by way of Examples and Comparative Examples, although the invention is not limited by these Examples. The quantitative determination methods carried out in the Examples are described below.

### Determination of Constituent Sugars (Glucose, Xylose and Mannose) and Klason Lignin

The test material such as pulp in an amount of 0.1 g was immersed for 2 hours in 1.5 mL of 72 wt% sulfuric acid at room temperature (25°C), following which 56 mL of water was added so as to dilute the sulfuric acid concentration to 3 wt% and the diluted solution was heated for 30 minutes at 121°C. The treated material was then filtered, separating it into an acid hydrolyzate liquor and a residue, following which the acid hydrolyzate liquor was neutralized by passing it through a Dionex OnGuard II A cartridge (Thermo Scientific), and the constituent sugars of the pulp were quantitatively determined by ion chromatography under the following conditions.

| | |
|---|---|
| • Apparatus: | Prominence ion analysis system LC-20ADsp (Shimadzu Corporation) |
| • Column: | CarboPac^{™} PA1 (4 × 250 mm; Thermo Scientific, Waltham, MA, USA) |
| • Detector: | DECADE Elite (Antec Scientific) |
| • Mobile phase: | 0.03 mol/L aqueous sodium hydroxide solution |
| • Flow rate: | 1.0 mL/min |
| • Column temperature: | 35°C |

In a separate procedure, the residue was dried within a thermostatic chamber at 105°C, and the constant weight was measured as the amount of Klason lignin.

### Determination of Metal Ions and Saturation Metal Amount in Pulp

The amount of metal ions included in the pulp following immersion treatment (metal content) was determined by ashing 1 g of pulp following the immersion step and then carrying ICP emission spectroscopy (ICPS-8100CL, from Shimadzu Corporation).

The saturation metal content of the pulp was the total amount of sodium ions, potassium ions, magnesium ions and calcium ions contained in the pulp obtained by immersing a pulp sample (length, 4 cm; width, 1 cm; thickness, 0.2 mm; weight, 1 g) for 18 hours in 100 mL of a 0.05 mol/L aqueous solution of sodium acetate or potassium acetate (liquid temperature, 25°C) as the aqueous monovalent metal salt solution and then washing the pulp with deionized water, followed in turn by suction filtration, drying in an oven (105°C, 24 hours), returning the pulp to normal temperature (20°C) and air drying. The average of the total content when using a 0.05 mol/L aqueous sodium acetate solution as the aqueous monovalent metal salt solution and when using a 0.05 mol/L aqueous potassium acetate solution was treated as the saturation metal content of the pulp.

Table 1 shows the details (proportions of constituent sugars and Klason lignin, and saturated metal content of pulp) on the pulp and other test materials used in Examples 1 to 4 and Comparative Examples 1 to 4.

**Table 1**

| Test material | Type of wood | Bleaching treatment | Constituent sugars (%) | | | Klason lignin (wt%) | Saturation metal amount in pulp (µmol/g) |
|---|---|---|---|---|---|---|---|
| | | | Glucose | Xylose | Mannose | | |
| Bleached Pulp 1 | softwood | yes | 87.7 | 8.7 | 3.6 | not detected (<0.5 wt%) | 19.1 |
| Bleached Pulp 2 | hardwood | yes | 83.8 | 16.2 | 0 | not detected (<0.5 wt%) | 24.1 |
| Bleached Pulp 3 | softwood | yes | 99.6 | 0.4 | 0 | not detected (<0.5 wt%) | 7.5 |
| Japanese cedar wood flour | softwood | no | 46.6 | 5.4 | 7.5 | 41.2 | - |
| Holocellulose | softwood | no | 71.9 | 7.7 | 8.5 | 7.9 | - |

The Japanese cedar wood flour is wood from the Japanese cedar (*Cryptomeria japonica*) that has been finely ground into flour and then deresinated.

The holocellulose was prepared as follows. First, 2.5 g of deresinated Japanese cedar wood flour was placed in a 300 mL Erlenmeyer flask and a 0.2 M acetic acid buffer solution (pH 3.5) was added, after which 1 g of sodium chlorite and 0.2 mL of acetic acid were added and 1 hour of heat treatment was carried out at 75°C under gentle stirring. Treatment consisting of, without cooling, the addition of 1 g of sodium chlorite and 0.2 mL of acetic acid to this reaction solution and heating for 1 hour was repeated three times, giving a holocellulose of Japanese cedar. This holocellulose was washed successively with 500 mL of distilled water and 50 mL of acetone, after which it was air dried and then dried in a 105°C oven, giving the test material.

### Determination of Levoglucosan in Examples 1 to 4 and Comparative Examples 1 to 4

The levoglucosan in Examples 1 to 4 and Comparative Examples 1 to 4 was quantitatively determined by ¹H-NMR under the following conditions.

| | |
|---|---|
| • Sample solvent: | 5 mg of an oximation reagent (NH₂OH·HCl) and 0.7 mL of heavy dimethylsulfoxide to which one drop of heavy water has been added |
| • Internal standard: | 0.46 mg of maleic acid |
| • Apparatus: | Avance III 400 NMR spectrometer from BRUKER |

### Determination of Carbon Monoxide, Methane and Hydrogen

Gases that formed in the heating step (carbon monoxide, methane and hydrogen) were quantitatively determined by gas chromatography under the following conditions.

| | |
|---|---|
| • Apparatus: | CP-4900 Micro GC, from Varian |
| • Column: | CP-Molsieve 5Å (length, 10 m; inside diameter, 0.32 mm; membrane thickness, 0.12 µm; from Agilent Technologies) |
| • Detector: | Micro-thermal conductivity detector (µTCD) |
| • Carrier gas: | Argon, 15 mL/min |
| • Column temperature: | 100°C |

### Determination of Carbon Dioxide and Ethylene

Gases that formed in the heating step (carbon dioxide and ethylene) were quantitatively determined by gas chromatography under the following conditions.

| | |
|---|---|
| • Apparatus: | CP-4900 Micro GC, from Varian |
| • Column: | CP-PoraPLOT Q (length, 10 m; inside diameter, 0.32 mm; membrane thickness, 0.10 µm; from Agilent Technologies) |
| • Detector: | Micro-thermal conductivity detector (µTCD) |
| • Carrier gas: | Helium, 15 mL/min |
| • Column temperature: | 80°C |

The value obtained by adding together the amounts of carbon monoxide, methane, hydrogen, carbon dioxide and ethylene was treated as the total amount of gas.

### Example 1

Bleached Pulp 1 (length, 4 cm; width, 1 cm; thickness, 0.2 mm; weight, 1 g; glucose content in constituent sugars, 87.7 wt%) was immersed in 100 mL of a 0.05 mol/L aqueous solution of magnesium acetate (solution temperature, 25°C) for 18 hours (immersion step). Next, the pulp was washed with deionized water, suction filtered, dried in an oven (105°C, 24 hours), returned to 20°C and air-dried, thereby producing immersion-treated pulp.

Next, 30 mg of the immersion-treated pulp was heated in an infrared furnace (the RHL-E45N Infrared Gold Image Furnace, from ADVANCE Riko Inc.; lamp voltage, 100 V; power, 4.0 kW; heating length, 140 mm; tube inside diameter, 52 mm) while passing nitrogen through the reaction tube in the infrared furnace (heating step). The infrared furnace had an output power of 1 kW, a heating time of 20 seconds, and a nitrogen linear velocity of 2.4 m/min). A gas-sampling bag (5-liter Tedlar bag) containing 30 mL of methanol was attached to the outlet of the reaction tube in the infrared furnace.

Because film-like char was found to have formed at the interior of the infrared furnace following completion of the heating step and carbon monoxide was not detected, the heating step is thought to have been carried out at between 400°C and 500°C. Also, given that film-like char had formed with a heating time of 20 seconds, the temperature ramp-up rate is thought to have been at least 50°C/s.

The gaseous product that formed with heating was cooled by the nitrogen flow, becoming an aerosol, which was collected by the gas-sampling bag (recovery step). This was left at rest for 30 minutes, following which 5 mL of neon gas was added to the bag as an internal standard, and gas identification and determination were carried out by gas chromatography.

Next, the methanol in which the product had dissolved was recovered, and the product which condensed on the inside walls of the sampling bag was dissolved in 200 mL of methanol and recovered. Using an evaporator, methanol was removed from 10 mL of the recovered methanol solutions, giving the solvent-extracted component.

The resulting solvent-extracted component was dissolved in 0.7 mL of heavy dimethylsulfoxide, and was confirmed by ¹H-NMR to be levoglucosan. The yield was 51.1 parts by weight per 100 parts by weight of glucose.

### Example 2

Aside from using Bleached Pulp 2 having a glucose content in the constituent sugars of 83.8 wt%, levoglucosan was obtained by carrying out the same operations as in Example 1. The yield of the resulting levoglucosan was 53.8 parts by weight per 100 parts by weight of glucose. Following completion of the heating step, a film-like char was found to have formed within the infrared furnace.

### Example 3

Aside from using a 0.05 mol/L aqueous calcium acetate solution as the aqueous divalent metal salt solution, levoglucosan was obtained by carrying out the same operations as in Example 1. The yield of the resulting levoglucosan was 42.4 parts by weight per 100 parts by weight of glucose. Following completion of the heating step, a film-like char was found to have formed within the infrared furnace.

### Example 4

Aside from using Bleached Pulp 3 having a glucose content in the constituent sugars of 99.6 wt%, levoglucosan was obtained by carrying out the same operations as in Example 1. The yield of the resulting levoglucosan was 46.6 parts by weight per 100 parts by weight of glucose. Following completion of the heating step, a film-like char was found to have formed within the infrared furnace.

### Comparative Example 1

Aside from using a 0.05 mol/L aqueous sodium acetate solution as the aqueous monovalent metal salt solution instead of the aqueous divalent metal salt solution in Example 1, levoglucosan was obtained by carrying out the same operations as in Example 1. The yield of the resulting levoglucosan was 7.3 parts by weight per 100 parts by weight of glucose. Following completion of the heating step, a film-like char was found to have formed within the infrared furnace.

### Comparative Example 2

Aside from not carrying out an immersion step, levoglucosan was obtained by carrying out the same operations as in Example 1. The yield of the resulting levoglucosan was 19.1 parts by weight per 100 parts by weight of glucose. Following completion of the heating step, a film-like char was found to have formed within the infrared furnace.

### Comparative Example 3

Aside from using Japanese cedar wood flour having a glucose content in the constituent sugars of 46.6 wt%, levoglucosan was obtained by carrying out the same operations as in Example 1. The yield of the resulting levoglucosan was 6.7 parts by weight per 100 parts by weight of glucose. Following completion of the heating step, a film-like char was found to have formed within the infrared furnace.

### Comparative Example 4

Aside from using holocellulose having a glucose content in the constituent sugars of 71.9 wt%, levoglucosan was obtained by carrying out the same operations as in Example 1. The yield of the resulting levoglucosan was 12.4 parts by weight per 100 parts by weight of glucose. Following completion of the heating step, a film-like char was found to have formed within the infrared furnace.

Table 2 presents the results from Examples 1 to 4 and Comparative Examples 1 to 4.

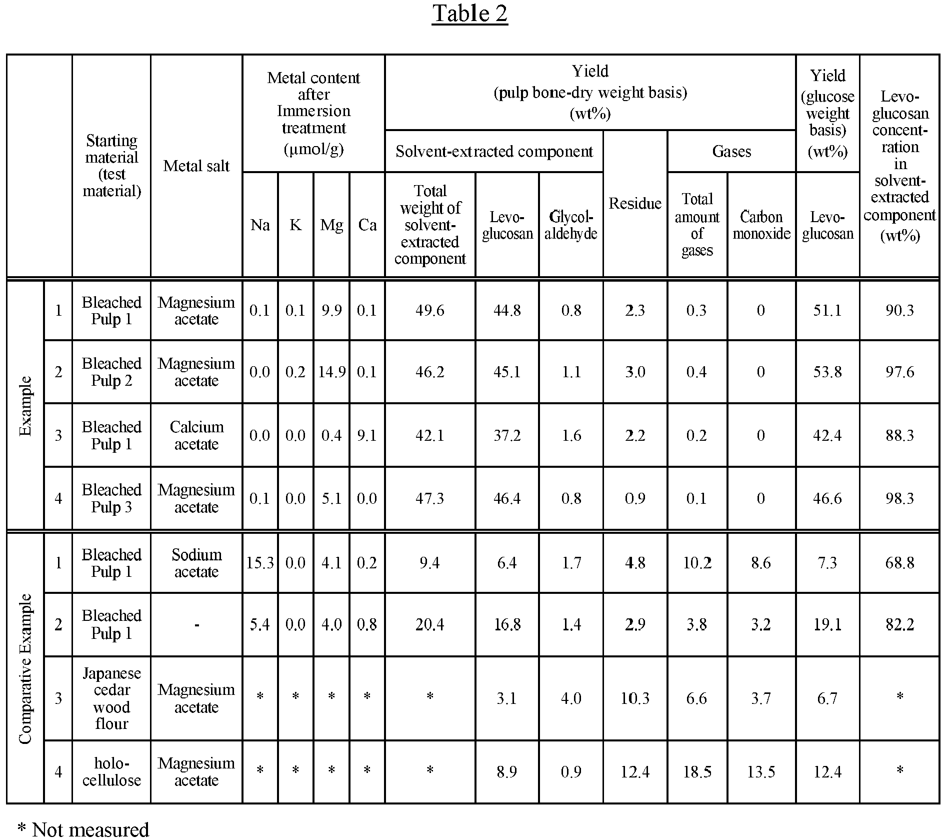

The results in Examples 1 to 4 and Comparative Example 2 demonstrate that, by carrying out immersion treatment prior to the heating step, levoglucosan can be easily obtained from pulp (cellulose) in a high yield and at high purity (levoglucosan concentration in solvent-extracted component). Also, in Examples 1 to 4, immersion-treated pulps in which divalent metal ions had formed coordination bonds with carboxyl groups in the pulp were used, as a result of which a film-like char formed and carbon monoxide did not form. Moreover, as explained above, we have confirmed that the weight loss temperature region in thermogravimetric (TG) measurement is about 50°C lower for immersion-treated pulp than for pulp containing monovalent metal ions. Considering this fact together with the subsequently described results of pulp temperature measurements during heat treatment of the pulp following immersion treatment, we estimated pyrolysis to have occurred at between 400°C and 450°C in the heating step. As a result, it appears that pulp pyrolysis can be promoted while suppressing low-selectivity pyrolysis reactions in the course of temperature ramp-up and also suppressing carbon monoxide formation at 500°C and above.

### Pulp Temperature Measurement During Heat Treatment of Pulp following Immersion Treatment

Using methods similar to the heating and temperature measurement methods and the evaluation method described in J. Anal. Appl. Pyrolysis (2014), 109, pp. 185-195, Bleached Pulp 3 following immersion treatment using magnesium acetate was heated for 50 seconds in a high-frequency heating furnace under a nitrogen atmosphere, and the temperature of the Bleached Pulp 3 was measured with thermocouples. Those results are shown in FIG. 1.

In the temperature rise chart of FIG. 1, the pulp temperature was raised from about 100°C to about 650°C in 10 seconds to 25 seconds after the start of temperature rise. During this temperature rising process, the rate of rise in the pulp temperature decreased slightly in the temperature range of 400 to 450°C, and so heat absorption associated with pyrolysis was confirmed. This demonstrates that pyrolysis of the pulp has occurred between 400 and 450°C. The surface of the char that formed liquefied under heating and became film-like.

## Claims

1. A method for producing levoglucosan, comprising the steps of:
(1) immersion-treating pulp having a content of glucose as a constituent sugar of at least 80 wt% and a Klason lignin content of not more than 1 wt% in an aqueous solution of a divalent metal salt;
(2) carrying out heat treatment by heating and thereby pyrolyzing the immersion-treated pulp to form gaseous levoglucosan and also to either not form char or form a film-like char and to either not form carbon monoxide or form not more than 5 parts by weight of carbon monoxide per 100 parts by weight (bone-dry weight) of the pulp; and
(3) recovering the gaseous levoglucosan by cooling it to a temperature at or below the boiling point of levoglucosan.

2. The method of claim 1, wherein the pulp has a content of xylose as a constituent sugar of from 5 to 20 wt%.

3. The method of claim 1 or 2, wherein the pulp has a content of glucose as a constituent sugar of not more than 95 wt%.

4. The method of any one of claims 1 to 3, wherein the divalent metal salt is a magnesium salt.

5. The method of any one of claims 1 to 4, wherein the pulp prior to immersion treatment has not been desalted.

6. The method of any one of claims 1 to 5, wherein the heat treatment is heating by infrared light irradiation.

7. The method of any one of claims 1 to 6, wherein the pulp heating time in heat treatment is at most 60 seconds.

8. The method of any one of claims 1 to 7, wherein the pulp itself has a temperature in the heat treatment step which is between 400°C and 450°C.
